(19) Europäisches Patentamt
European Patent Office
Office européen des brevets

(11) EP 1 733 711 A1

(12) EUROPEAN PATENT APPLICATION
published in accordance with Art. 158(3) EPC

(43) Date of publication:
**20.12.2006 Bulletin 2006/51**

(21) Application number: **05721169.0**

(22) Date of filing: **18.03.2005**

(51) Int Cl.:
***A61K 8/18*** *(2006.01)*

(86) International application number:
**PCT/JP2005/005019**

(87) International publication number:
**WO 2005/089701 (29.09.2005 Gazette 2005/39)**

(84) Designated Contracting States:
**DE FR GB**

(30) Priority: **19.03.2004 JP 2004079724**
**19.03.2004 JP 2004081328**

(71) Applicant: **KAO CORPORATION**
**Tokyo 103-8210 (JP)**

(72) Inventors:
- **YOSHIDA, Kensuke**
**c/o Kao Corp., Research Lab.**
**Tokyo 1318501 (JP)**
- **OKUDA, Shinsuke**
**c/o Kao Corp., Research Lab.**
**Tokyo 1318501 (JP)**
- **NAKAMURA, Sugiko**
**c/o Kao Corp., Research Lab.**
**Wakayama 6408580 (JP)**
- **NAMBU, Hiromi**
**c/o Kao Corp., Research Lab.**
**Wakayama 6408580 (JP)**
- **KUWAHARA, Kazuo**
**c/o Kao Corp., Research Lab.**
**Wakayama 6408580 (JP)**

(74) Representative: **HOFFMANN EITLE**
**Patent- und Rechtsanwälte**
**Arabellastrasse 4**
**81925 München (DE)**

# (54) COSMETIC PREPARATION FOR EYELASH

(57) The present invention relates to a cosmetic for eyelash comprising a film-forming polymer (a) having a rate of change in polymer content (ΔW) of 13% or more, and water, wherein a content of the film-forming polymer (a) is 0.6 to 50% by mass. The cosmetic for eyelash according to the present invention is free from formation of lumps upon use, and imparts a good curl effect to the eyelash.

EP 1 733 711 A1

## Description

### TECHNICAL FIELD

The present invention relates to cosmetics for eyelash containing a film-forming polymer which are free from formation of lumps upon use and simply impart a good curl effect to eyelashes.

### BACKGROUND ART

In order to enhance cosmetic effects of mascara for making eyes attractive, its capability for curling eyelashes upwardly, namely curl-up effect thereof is of significant importance. Conventionally, to enhance the curl-up effect of mascara, there has been used such a method in which a drying speed of a bulk of mascara is promoted such that the mascara is dried up during application thereof to prevent eyelashes lifted with a mascara brush from hanging down and maintain the eyelashes in upwardly curled state. In general, to attain a high drying speed of the mascara bulk, a quick-drying film-forming material or a solvent having a high volatilizing speed has been used in the mascara composition.
However, in the above method, the quick-drying mascara tends to form a coating film during application thereof, so that eyelashes repeatedly coated with such a mascara in a wet-on-wet manner tend to frequently suffer from considerably poor appearance upon finishing. Namely, since the mascara is dried up during application thereof, there tends to arise such a problem lumps are formed on eyelashes treated therewith.
To solve these problems, there have been proposed mascara compositions prepared by using a specific film-forming polymer in combination with various kinds of solid particles to impart a good curl effect to eyelashes. For example, JP 11-256619A has proposed a mascara composition obtained from combination of a film-forming polymer capable of causing stratum corneum to be contracted more than 1% and a high-hardness wax for exhibiting both a curl effect and an effect of increasing a concentration of color of eyelashes. In addition, JP 2003-55136A, JP 2003-55156A, JP 2003-55157A and JP 2003-55158A have proposed a mascara composition obtained from combination of a polymer adherable to a keratinous substance and particles or waxes of the other polymer to impart a good curl effect to eyelashes.
However, the conventional mascara compositions still tend to have problems such as formation of lumps on eyelashes upon use.
Also, with the application of the conventional mascara compositions, an eyelash curler have been actually frequently used to impart a more excellent curl effect to eyelashes. However, the use of eyelash curler requires a skill, consumes time, and takes a larger labor of the user, while the eyelashes are damaged and pulled off. In other word, the conventional mascara compositions have required auxiliary tools such as the eyelash curler, and therefore fail to attain a sufficient curl effect by themselves.

### DISCLOSURE OF THE INVENTION

The present invention relates to a cosmetic for eyelash which is free from formations of lumps even when repeatedly coating eyelashes therewith in a wet-on-wet manner and is capable of simply imparting a good curl effect to eyelashes.
The inventors have found that such a cosmetic for eyelash prepared by using a film-forming polymer having a specific contraction in combination with a volatile component such as water can be prevented from suffering from formation of lumps upon use and can impart a good curl effect to eyelashes.
Thus, the present invention provides a cosmetic for eyelash including a film-forming polymer (a) having a rate of change in polymer content ($\Delta W$) of 13% or more, and water, wherein a content of the film-forming polymer (a) in the cosmetic being from 0.6 to 60% by mass. Further, the present invention provides a cosmetic including (A) 10% by mass or more of a non-volatile component and (B) 30% by mass or more of a volatile component wherein the non-volatile component (A) contains (a-1) 70% by mass or more of a non-volatile component kept in a solid state at ordinary temperature which contains the film-forming polymer (a), and (a-2) 30% by mass or less of a non-volatile component kept in a liquid state at ordinary temperature.
The cosmetic for eyelash according to the present invention is free from formation of lumps upon use even when repeatedly coating eyelashes therewith in a wet-on-wet manner and is capable of imparting a good curl effect to eyelashes and attaining a sufficiently high curl effect without using tools such an eyelash curler, etc.

### PREFERRED EMBODIMENTS FOR CARRYING OUT THE INVENTION

The cosmetic for eyelash according to the present invention contains a film-forming polymer (a) having a rate of change in polymer content ($\Delta W$) of 13% or more in an amount of from 0.6 to 50% by mass on the basis of a total amount of the cosmetic. Here, the rate of change in polymer content ($\Delta W$) has the following definition:

(Definition of Rate of Change in Polymer Content (ΔW): Film-Forming Test)

ΔW is a rate of change in polymer content which is caused during a film-forming process in which a film obtained from the polymer which has a pencil hardness of 2B is dried up, said ΔW being represented by the following formula (I):

$$\Delta W = W2 - W1 \qquad (I)$$

wherein W1 and W2 are respectively defined as follow:

An aqueous solution containing 20% by mass of the polymer is applied onto two glass plates over an area of 11 cm x 20 cm thereof using an applicator to form a coating film having 250 μm, and then dried at a temperature of 23°C and a relative humidity (RH) of 65%; and the first coating film formed by spreading the solution on one glass plate is subjected to measurement of change in weight thereof upon drying, and the second coating film formed on another glass plate is subjected to measurement of a hardness thereof by a pencil hardness measuring method to calculate a polymer content W1 (%) upon drying the coating film into a pencil hardness of 2B (hereinafter occasionally referred to as "upon solidifying") according to the following formula, and further the coating film is allowed to stand for 24 h after coating (hereinafter referred to as "after drying up") to calculate a polymer content W2 (%) after drying up according to the following formula:

$$W1\ (\%) = (Wp/W1t) \times 100$$

$$W2\ (\%) = (Wp/W2t) \times 100$$

wherein Wp represents a weight of the polymer in the coating film upon coating; W1t represents a weight of the coating film upon drying into a pencil hardness of 2B; and W2t is a weight of the coating film after drying up.

The method of measuring the pencil hardness as used in the above definition is as follows.

(Method of Measuring Pencil Hardness)

The measurement of the pencil hardness is conducted according to JIS K 5400. In the measurement, a solution of the polymer is applied onto a glass plate using an applicator to form a coating film having a thickness of 250 μm, and then dried at a temperature of 23°C and a relative humidity of 65%. Thereafter, the thus obtained coating film was placed in a horizontal plane, and a pencil was pressed thereagainst at a fixed angle of 45° under a load of 750 g to measure a pencil hardness thereof.

The cosmetic for eyelash according to a preferred embodiment of the present invention contains (A) 10% by mass or more of a non-volatile component and (B) 30% by mass or more of a volatile component wherein the non-volatile component (A) contains 70% by mass or more of a non-volatile component (a-1) kept in a solid state at ordinary temperature which contains the film-forming polymer (a), and 30% by mass or less of a non-volatile component (a-2) kept in a liquid state at ordinary temperature. Here, the non-volatile component means such a compound having a vapor pressure of less than 10 Pa at ordinary temperature (25°C) under normal pressures.

The non-volatile component (a-1) kept in a solid state at ordinary temperature contains the film-forming polymer (a) having a rate of change in polymer content (ΔW) of 13% or more as described above. The film-forming polymer (a) having a rate of change in polymer content (ΔW) of 13% or more can contain a large amount of a volatile solvent upon initiation of solidification thereof. For this reason, the cosmetic for eyelash containing such a polymer can be easily applied and is free from formation of lumps even when repeatedly coating eyelashes therewith in a wet-on-wet manner. Further, it is considered that upon applying the cosmetic for eyelash onto eyelashes to form a coating film thereon, the eyelashes can be efficiently curled by contraction of a volume of the resultant coating film due to drying caused for a period of from the "upon solidifying" to the "after drying up".

The upper limit of the rate of change in polymer content (ΔW) of the film-forming polymer (a) is preferably 35% or lower in view of preventing eyelashes from suffering from a too large curl. The rate of change in polymer content (ΔW) of the film-forming polymer (a) is more preferably 15 to 30% in view of imparting a suitable curl to eyelashes.

Also, the film-forming polymer (a) used in the present invention preferably has a weight-average molecular weight of 5,000 to 2,000,000 in view of an adequate coating easiness and a sufficient curl effect. When the weight-average molecular weight of the film-forming polymer (a) is 5,000 or more, the resultant cosmetic exhibits a sufficient curl effect, whereas when the weight-average molecular weight of the film-forming polymer (a) is 2,000,000 or less, the resultant cosmetic exhibits a good feeling upon use. In view of both feeling upon use and curl effect, the weight-average molecular weight of the film-forming polymer (a) is preferably 8,000 or more, more preferably 20,000 or more and still more preferably 50,000 or more, and also preferably 1,000,000 or less, more preferably 500,000 or less and still more preferably 300,000 or less.

Examples of the suitable film-forming polymer (a) include vinyl-based (co)polymers and urethanes such as polyurethane, acryl-polyurethanes, polyester-polyurethanes and polyether-polyurethanes.

Among these polymers, as the vinyl-based (co)polymers , preferred are copolymers obtained by copolymerizing a vinyl monomer, and more preferred are polymers obtained by polymerizing a monomer containing a reactive vinyl group-containing organic acid and/or a salt thereof.

The reactive vinyl group-containing organic acid is a water-soluble organic acid containing one or more reactive vinyl groups and one or more acid groups in a molecule thereof. Specific examples of the reactive vinyl group-containing organic acid include unsaturated carboxylic acid monomers such as acrylic acid, methacrylic acid, crotonic acid, itaconic acid and maleic acid; unsaturated sulfonic acid monomers such as styrenesulfonic acid, 2-acrylamido-2-methylpropanesulfonic acid, 3-sulfopropyl (meth)acrylic esters, bis((3-sulfopropyl)itaconic esters and vinylsulfonic acid; and unsaturated phosphoric acid monomers such as vinyl phosphate, bis((meth)acryloxyethyl)phosphate, diphenyl-2-(meth)acryloyloxyethyl phosphate, dibutyl-2-(meth)acryloyloxyethyl phosphate and dicotyl-2-(meth)acryloyloxyethyl phosphate. These reactive vinyl group-containing organic acids may be used alone or in the form of a mixture of any two or more thereof.

Among the monomers containing the above reactive vinyl group-containing organic acid and/or the salt thereof, in view of enhancing a curl effect of eyelashes, preferred are unsaturated carboxylic monomers and salts thereof, and unsaturated sulfonic acid monomers and salts thereof. Among the unsaturated carboxylic monomers having a relatively low acidity and salts thereof, more preferred are methacrylic acid and salts thereof. Among the unsaturated sulfonic acid monomers and salts thereof, more preferred are styrenesulfonic acid and salts thereof.

As the salt of the reactive vinyl group-containing organic acid, there may be used either inorganic salts or organic salts of the above organic acids. Examples of the inorganic salts include alkali metal salts such as Na salts and K salts, alkali earth metal salts and ammonium salts. Examples of the organic salts include salts of L-arginine and 2-aminomethyl-1-propanol. Among these salts, preferred are inorganic salts, and more preferred are ammonium salts.

Also, the film-forming polymer (a) is preferably partially or entirely neutralized in view of improving a water resistance and a curling property and preventing formation of lumps. More specifically, the degree of neutralization of the film-forming polymer (a) is preferably in the range of from 0.01 to 1.0, more preferably from 0.01 to 0.5 and still more preferably from 0.02 to 0.3.

In particular, among the above film-forming polymers, preferred are sodium salts and ammonium salts of polymethacrylic acid and polystyrenesulfonic acid, and more preferred are ammonium salts of these acids. Among the polymethacrylic acid and/or salts thereof, preferred are those having a weight-average molecular weight of 20,000 to 300,000, and more preferred are those having a weight-average molecular weight of 60,000 to 200,000. Among the polystyrenesulfonic acid and/or salts thereof, preferred are those having a weight-average molecular weight of 50,000 to 1,000,000, and more preferred are those having a weight-average molecular weight of 100,000 to 500,000.

The cosmetic for eyelash according to the present invention contains the film-forming polymer (a) in an amount of 0.6 to 50% by mass on the basis of a total amount of the cosmetic. When the content of the film-forming polymer (a) is 0.6% by mass or more, the resultant cosmetic exhibits a sufficient curl effect, whereas when the content of the film-forming polymer (a) is 50% by mass or less, the resultant cosmetic is easily usable in view of its viscosity. In order to impart a good curl effect to eyelashes, the content of the film-forming polymer (a) in the cosmetic is preferably 1% by mass or more, more preferably 2% by mass or more, still more preferably 3% by mass or more and further still more preferably 3.5% by mass or more. Further, in view of preventing formation of lumps even upon repeatedly coating eyelashes with the cosmetic in a wet-on-wet manner and exhibiting a good curl effect, the film-forming polymer (a) is preferably contained in the cosmetic in an amount of 1 to 15% by mass. When the content of the film-forming polymer (a) in the cosmetic is 15% by mass or less, the resultant cosmetic exhibits a more suitable feeling upon use. From the above viewpoint, the content of the film-forming polymer (a) in the cosmetic is more preferably 10% by mass or less and still more preferably 6% by mass or less.

In the present invention, the non-volatile component (a-1) kept in a solid state at ordinary temperature preferably contains a wax in addition to the above film-forming polymer (a). The wax used herein may be appropriately selected from animal waxes, vegetable waxes, mineral waxes and synthetic waxes. Specific examples of the wax include carnauba wax, candelilla wax, rice wax, beeswax, extremely hydrogenated jojoba oil, lanolin wax, microcrystalline wax, ceresin, paraffin wax, polyethylene wax, glycerides kept in a solid state at ordinary temperature (25°C) and silicone wax.

Among these waxes, in view of achieving an especially higher curl-up effect, more preferred are waxes having a rate of penetration of 8 or more. The rate of penetration used herein may be measured according to JIS K-2235-5.4. More specifically, the rate of penetration is expressed by the value which is 10 times a penetration distance (mm) obtained

by measuring a length of a portion of a specified needle (mass of needle: 2.5±0.02 g; mass of needle holder: 47.5±0.02 g; mass of weight: 50±0.05 g) which portion is penetrated into a sample wax maintained at 25°C±0.1°C for 5 s. Specific examples of the preferred wax having a rate of penetration of 8 or more include beeswax and microcrystalline wax.

The wax having a rate of penetration of 8 or more is preferably contained in the component (a-1) in an amount of 0.1 to 20% by mass. When the content of the wax having a rate of penetration of 8 or more in the component (a-1) is 0.1% by mass or more, the resultant cosmetic exhibits a sufficient curl-up effect of eyelashes. On the other hand, when the content of the wax having a rate of penetration of 8 or more in the component (a-1) is 20% by mass or less, the resultant cosmetic exhibits a good spreadability upon its application to eyelashes. In view of both good spreadability and sufficient curl effect, the content of the wax having a rate of penetration of 8 or more in the component (a-1) is preferably in the range of from 1 to 15% by mass and more preferably from 3 to 10% by mass.

In addition, a mass ratio of the film-forming polymer (a) to the wax having a rate of penetration of 8 or more (film-forming polymer (a)/wax) is preferably in the range of from 1/30 to 3/1 and more preferably from 1/5 to 1/1 in view of good beauty and good curl effect upon finishing.

In the present invention, the non-volatile component (a-1) kept in a solid state at ordinary temperature preferably contains a powder having an average particle size of 0.1 to 20 μm or a water dispersion thereof. When such a powder contained in the non-volatile component (a-1) has an average particle size of 0.1 μm or more, the resultant cosmetic exhibits a good touch feeling upon application thereof. Also, when the powder contained in the non-volatile component (a-1) has an average particle size of 20 μm or less, the resultant cosmetic is free from formation of lumps. In particular, the average particle size of the powder contained in the non-volatile component (a-1) is more preferably 0.2 to 10 μm and still more preferably 1 to 10 μm. The average particle size of the powder is a median diameter measured by a laser diffraction method using a laser diffraction/light-scattering particle size distribution measuring apparatus (for example, "LA-920" available from Horiba, Ltd.).

The powder contained in the non-volatile component (a-1) may have any suitable shape such as a spherical shape, a flat plate shape, a granular shape, an acicular shape, a bar shape and an amorphous shape. The powder may be either an inorganic powder or an organic powder.

Specific examples of the powder contained in the non-volatile component (a-1) include inorganic powders, e.g., plate-shaped inorganic powders such as talc, mica, sericite and kaolin, and spherical or amorphous inorganic powders such as silica, barium sulfate, aluminum hydroxide, calcium carbonate, magnesium silicate, magnesium carbonate and aluminum silicate; and organic powders such as polyamide resins, polyethylene resins, polypropylene resins, polymethyl methacrylate resins, cellulose-based resins, polystyrene resins, styrene-acrylic acid copolymers and silicone resins. Among these powders, preferred are inorganic powders, more preferred are spherical inorganic powders, and still more preferred is silica. Meanwhile, the organic powders may be hydrophilized to render a configuration thereof more suitable.

When the powder contained in the non-volatile component (a-1) is used in the form of a water dispersion thereof, the powder may be a water-dispersible hydrophilic powder. When the powder is hydrophobic, the powder may be dispersed in water using a water-soluble polymer or a surfactant to form a water dispersion thereof.

Specific examples of the water dispersion include a polyethylene dispersion, a silicone resin dispersion, a polystyrene resin dispersion, an urethane dispersion and a nylon dispersion.

These powders may be used alone or in the form of a mixture of any two or more thereof. In order to attain a high curl effect without formation of lumps, the component (a-1) preferably contains the powder in an amount of 0.1 to 20% by mass. When the content of the powder in the component (a-1) is 0.1% by mass or more, the resultant cosmetic exhibits a sufficient curl-up effect. Also, when the content of the powder in the component (a-1) is 20% by mass or less, the resultant cosmetic can be effectively prevented from suffering from formation of lumps. The mass ratio of the film-forming polymer (a) to the powder (film-forming polymer (a)/powder) is preferably in the range of from 1/5 to 10/1 and more preferably from 1/2 to 2/1 in view of preventing formation of lumps and exhibiting a high curl-up effect.

The cosmetic for eyelash according to the present invention may also contain a pigment. The pigment is not particularly limited as long as it can be used in ordinary cosmetic compositions. Examples of the pigment include inorganic pigments and organic pigments.

Specific examples of the inorganic pigments include titanium oxide, black titanium oxide, zinc white, red iron oxide, yellow iron oxide and black iron oxide. Specific examples of the organic pigments include tar pigments. The pigment is preferably contained in an amount of 0.1 to 20% by mass and more preferably 1 to 8% by mass on the basis of a total amount of the cosmetic for eyelash.

The powder and the pigment may be subjected to hydrophilic treatments such as silica treatment, alumina treatment, silica-alumina treatment and polyacrylic acid treatment, and hydrophobic surface treatments such as silicone treatment, fluorine compound treatment, metal soap treatment, lecithin treatment and oils and fats treatment. Among these treatments, in view of a good curl effect and a good appearance upon finishing, more preferred are hydrophilic treatments, and most preferred is the polyacrylic acid treatment.

Further, the component (a-1) may also contain, in addition to the above film-forming polymer (a), the other film-forming polymer or a film-non-forming polymer unless the addition thereof adversely affects the effects of the present invention. The other film-forming polymer or the film-non-forming polymer may be added as a thickening agent, a dispersion stabilizer or a film-forming assistant. Examples of the other film-forming polymer or the film-non-forming polymer include emulsions of polymers such as homopolymers or copolymers of alkyl esters of acrylic acid or methacrylic acid, alkyl acrylate-styrene copolymers and polyvinyl acetate; polyvinyl alcohol; silicone-based polymer; natural substances such as guar gum, gum arabic, sodium alginate, carageenan, xanthan gum and starches; and semi-synthetic substances such as modified cone starches, methyl cellulose, hydroxyethyl cellulose, hydroxypropyl cellulose, carboxymethyl cellulose and cationized hydroxyethyl cellulose. These polymers may be used in the form of any of a solution and an emulsion. In particular, in order to impart a good water resistance or a good moisture resistance to the cosmetic, the polymers may be blended in the form of an emulsion.

In addition, in order to attain a good long lash effect, the component (a-1) may also contain a fiber. As the fiber, there may be used any of natural fibers such as cotton, silk and hemp, regenerated fibers such as rayon, and synthetic fibers such as polyamides, polyesters, acrylic resins and polyolefins. In view of a good strength, among these fibers, preferred are polyamide fibers such as nylons. If required, the fiber may be surface-treated. Examples of the surface treatment include a silica treatment, a silicone treatment, a fluorine compound treatment, a metal soap treatment and an oils and fats treatment. Also, in view of good adhesion to eyelashes, the fiber preferably has a fineness of 0.1 to 20T and a length of 0.1 to 5 mm. The content of the fiber in the component (a-1) is preferably 0.1 to 6% by mass on the basis of a total amount of the cosmetic in view of attaining a good long lash effect.

In the present invention, the non-volatile component (a-2) kept in a liquid state at ordinary temperature preferably includes liquid oils and/or polyols which may be used in ordinary cosmetic compositions.

The non-volatile component kept in a liquid state at ordinary temperature may be a liquid oil component which is non-volatilized at ordinary temperature (25˚C) under normal pressures. Examples of the liquid oil component include hydrocarbon-based oils such as light isoparaffins, liquid isoparaffins, liquid paraffins and heavy liquid isoparaffins; esters or triglycerides such as diisostearyl malate, isotridecyl isononanoate, glyceryl dimyristate, glyceryl diisostearate, glyceryl myristate isostearate, neopentyl glycol dicaprate, castor oil, macadamia nut oil and jojoba oil; and silicone oils such as dimethyl polysiloxane, methylphenyl polysiloxane, dimethyl cylcopolysiloxane and methyl hydrogen polysiloxane.

Examples of the polyols include ethylene glycol, diethylene glycol, polyethylene glycol, polypropylene glycol, propylene glycol, dipropylene glycol, 1,3-butylene glycol, polyethylene glycol and glycerol. Among these polyols, in view of easiness of use, preferred are glycerol and 1,3-butylene glycol, and further in view of preventing formation of lumps, more preferred is 1,3-butylene glycol.

Also, the component (a-2) may contain a surfactant. Examples of the surfactant include anionic surfactants, cationic surfactants, nonionic surfactants and amphiphilic surfactants. These surfactants may be used alone or in combination of any two or more thereof. Among these surfactants, preferred are those having a melting point of 25˚C or lower.

The non-volatile component (A) used in the present invention preferably contains the above component (a-1) in an amount of 70% by mass or more and the above component (a-2) in an amount of 30% by mass or less. When the content of the component (a-1) in the non-volatile component (A) is 70% by mass or more, the resultant cosmetic imparts a sufficient curl effect to eyelashes.

Further, the cosmetic of the present invention preferably contains the volatile component (B) in an amount of 30% by mass or more on the basis of a total amount of the cosmetic.

The volatile component use herein means a compound having a vapor pressure of 10 Pa or higher at ordinary temperature (25˚C) under normal pressures. Examples of the volatile component include water, lower alcohols, volatile hydrocarbon oils and volatile silicones.

Examples of the lower alcohols include alcohols having 1 o 4 carbon atoms such as ethanol, propanol, isopropanol, butyl alcohol and isobutyl alcohol. Examples of the volatile hydrocarbon oils include hydrocarbon oils having 8 to 16 carbon atoms, in particular, petroleum-derived hydrocarbon oils such as isoparaffins (light isoparaffins) and isododecane (2,2,4,4,6-pentamethyl heptane). Examples of the volatile silicones include volatile ones of linear or cyclic silicones represented by the following formula (1) or (2):

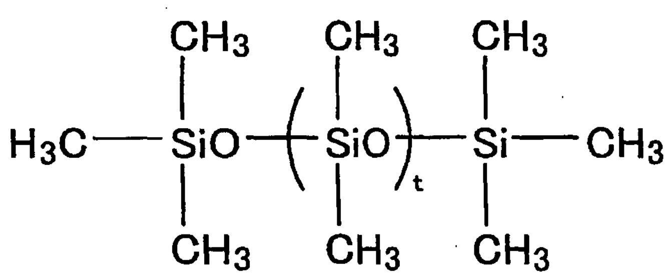

(1)

wherein t is an integer of 0 to 3, or

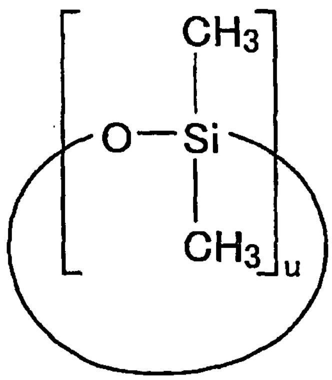

(2)

wherein u is an integer of 3 to 5.

Specific examples of the silicone oils include octamethyl trisiloxane, decamethyl tetrasiloxane, dodecamethyl cyclohexasiloxane, octamethyl cyclotetrasiloxane and decamethyl cyclopentasiloxane.

These volatile components may be used alone or in the form of a mixture of any two or more thereof. The content of the volatile component is 30% or more. In particular, the volatile component preferably contains water as a volatile solvent. The content of a volatile solvent other than water in the volatile component is preferably 0.5 to 20% by mass. The volatile solvent other than water is preferably a lower alcohol having 1 to 4 carbon atoms and more preferably ethanol in view of easiness of handling.

Meanwhile, the mass ratio of the film-forming polymer (a) to ethanol (film-forming polymer (a)/ethanol) is preferably in the range of from 1/10 to 20/1 and more preferably from 1/4 to 5/1 in view of a good contraction of the polymer and a good curl-up effect.

The volatile component (B) of the cosmetic for eyelash according to the present invention may be in the form of a water-containing emulsions such as an oil-in-water (O/W) type emulsion system and a water-in-oil (W/O) type emulsion system. Among these systems, in view of preventing formation of lumps and enhancing a curl-up effect, preferred is a system containing water as the volatile component (B), more preferred is a system containing water and a lower alcohol having 1 to 4 carbon atoms as the volatile component (B), and most preferred is an oil-in-water (O/W) type emulsion system using these compounds.

The cosmetic for eyelash according to the present invention preferably exhibits an angle of bend of 5° or more, more preferably 10° or more and still more preferably 15° or more when 0.04 g of the cosmetic is applied onto a polyethylene terephthalate film ("Melinex S" available from DuPont Teijin Films; hereinafter referred to merely as "PET film") having a width of 2 cm, a length of 5 cm and a thickness of 75 $\mu$m to form a coating film having a width of 4 mm in a length direction of the polyethylene terephthalate film and a length of 2 cm in a width direction of the polyethylene terephthalate film, on a central portion of the length direction of the polyethylene terephthalate film, and dried at a temperature of 23°C and a relative humidity of 60% for 12 h. Meanwhile, a maximum value of the angle of bent is 180°.

The cosmetic for eyelash according to the present invention may also contain in addition to the above components, various additive components blended in ordinary cosmetics for imparting a good cosmetic effect thereto unless the addition thereof adversely affects the effects of the present invention. Examples of the additive components include at least one of a thickening agent, an ultraviolet absorber, an ultraviolet scattering agent, a humectant, an antioxidant, a perfume and an antiseptic agent.

The cosmetic for eyelash according to the present invention preferably has a viscosity of 100 to 2000 Pa·s as measured at 25°C. When the viscosity of the cosmetic lies within the above-specified range, the cosmetic exhibits a good adhesion to eyelashes, imparts a good finishing appearance thereto, and further can be easily produced. The viscosity of the cosmetic is more preferably in the range of 150 to 1000 Pa·s as measured at 25°C.

The cosmetic for eyelash according to the present invention may be produced by ordinary methods, for example, by uniformly mixing the above respective components with each other under stirring.

The cosmetic for eyelash according to the present invention is intended to be used as a make-up cosmetic for eyelashes by blending a coloring pigment therein, more specifically, as mascara. Also, the cosmetic using no coloring pigment may also be used as a so-called primer or top coat for eyelashes.

When the cosmetic for eyelash according to the present invention is used as the make-up cosmetic, the cosmetic may be applied to eyelashes with a brush, etc., which may be used for ordinary mascara. In particular, a more excellent

curl effect can be attained by increasing an amount of the cosmetic adhering onto an upper surface of eyelash. For example, the cosmetic may be applied to eyelashes from above or repeatedly applied thereto from beneath using an ordinary coating tool for mascara such as a brush, a comb-shaped applicator, a coil-shaped applicator or paintbrush type, flocky, a bar-shaped applicator and a spatula-shaped applicator, so that a coating amount of the cosmetic applied onto the upper surface of eyelashes is increased as compared to that applied to a lower surface of eyelashes, resulting in a good curl effect.

In the cosmetic for eyelash according to the present invention containing the film-forming polymer (a) having a rate of change in polymer content (ΔW) of 13% or more in the above specified range, the film-forming polymer (a) not only acts as a thickening agent or a film-forming agent, but also imparts a good contraction to eyelashes, thereby attaining a good curl effect.

EXAMPLES

PRODUCTION EXAMPLE 1: Production of Polymethacrylic Acid

300 g of methacrylic acid available from Wako Pure Chemical Industries, Ltd., 1.5 liters of ethanol and 1.73 g of an initiator "V-65" available from Wako Pure Chemical Industries, Ltd., were charged into a glass reaction container, and polymerized at 65°C for 4 hours. The resultant polymer solution was dropped into 20 liters of acetone, and re-precipitated. The resultant precipitate was recovered and dried under reduced pressure at 65°C for 12 hours, thereby obtaining a polymethacrylic acid (PMAA). As a result of measuring a molecular weight of the thus obtained product by gel permeation chromatography (GPC), it was confirmed that the polymethacrylic acid had a molecular weight of 180,000 in terms of polyethylene glycol. Meanwhile, the pH value of the polymethacrylic acid was adjusted to 6 using sodium hydroxide. Also, it was confirmed that a rate of mass reduction (ΔW) of the polymethacrylic acid as measured during a period of from the time at which a hardness of a coating film prepared therefrom reached a pencil hardness of 2B to the time at which the coating film was completely dried up, was 21% by mass.

(1) Evaluation of Curl

Three hair fibers each having a length of 1 cm were sampled and fixed in a horizontal plane. Then, a mascara composition shown in Table 1 was applied onto the hair fibers with a brush 10 times in such a manner as to lift the hair fibers upwardly from beneath, and an angle of warpage of the hair fibers from the horizontal plane was measured. The measurement was conducted three times, and an average value of the three measured values was calculated. The curling of the hair fibers was evaluated from the average value according to the following evaluation criteria.

◎: 20° or more
○:15° or more but less than 20°
Δ: 10° or more but less than 10°
×: Less than 10°

(2) Evaluation of Adhesion

The hair fibers coated with the cosmetic in the same manner as in the above evaluation of curl were observed by naked eyes by expert panelists to evaluate the adhesion of the cosmetic thereto according to the following evaluation criteria.

◎: Very uniformly adhered
○: Uniformly adhered
Δ: Slightly uneven adhesion occurred
×: Uneven adhesion occurred

(3) Evaluation of Formation of Lumps

The prepared mascara composition was applied onto false eyelashes as a test specimen with a brush to observe the extent of formation of lumps thereon by naked eyes. The results were evaluated according to the following evaluation criteria.

◎: No formation of lumps
○: Slight formation of lumps but no significant influence on appearance upon finishing

Δ: Slight formation of lumps and some influence on appearance upon finishing
×: Significant formation of lumps

(4) Evaluation of Contraction

The cosmetic in an amount of 0.04 g was applied onto a polyethylene terephthalate (PET) film having a size of 2 cm x 5 cm (thickness: 75 μm) which was fixed at one surface thereof onto a slide glass with a tape, to form a strip-like coating film having a width of 4 mm in a length direction of the polyethylene terephthalate film, on a central portion of the width direction of the film, and dried at a temperature of 23˚C and a relative humidity of 60% for 12 hours to measure an angle of bend of the film using a protractor. The larger angle of bend indicates a higher and more excellent contraction. The PET film used was "Melinex S (tradename)" (thickness: 75 μm) available from DuPont Teijin Films. The measurement was conducted at 3 points per one sample, and an average value of the three measured values was calculated and regarded as an angle of bend of the film.

(5) Average Particle Size of Powder

The average particle size of the powder was determined as a median diameter measured by a laser diffraction method using a light-scattering particle size distribution measuring apparatus "LA-920" available from Horiba Ltd.

EXAMPLES 1 TO 5 AND COMPARATIVE EXAMPLES 1 AND 2

Using the polymer shown in Table 1 (whose ΔW was measured by the above-mentioned method), the cosmetics having compositions shown in Table 1 were prepared. The viscosity of each of the obtained cosmetics was measured at 23˚C and 6 r/min using a B-type viscometer, and the angle of curvature of a coating film prepared from the cosmetic was measured by the above method. Also, the cosmetic was subjected to a sensory evaluation with respect to a cosmetic effect of rendering eyes attractive (curl-up effect) and adhesion to eyelashes when used as a primer for mascara by the above-mentioned methods (1) and (2). The results are shown in Table 1.

TABLE 1

| | Examples | | | | | Comparative Examples | |
|---|---|---|---|---|---|---|---|
| | 1 | 2 | 3 | 4 | 5 | 1 | 2 |
| Kind of polymer and ΔW (%) | PMAA*1 21 | PMAA*1 21 | PMAA*1 21 | PNaSS*2 17 | PNaSS*2 17 | PVP*3 1 | HEC*4 1 |
| Composition of cosmetic (mass %) | | | | | | | |
| Polymer | 20 | 20 | 20 | 30 | 20 | 20 | 5 |
| Ethanol | 0 | 6 | 0 | 0 | 0 | 0 | 0 |
| Polyoxyethylene (16) octadodecyl ether | 0 | 0 | 0.01 | 0 | 0 | 0 | 0 |
| Water | Balance | Balance | Balance | Balance | Balance | Balance | Balance |
| Total | 100 | 100 | 100 | 100 | 100 | 100 | 100 |
| Viscosity of cosmetic (mPa·s) | 7000 | 6500 | 7000 | 520 | 30000 | 6500 | 15000 |
| Evaluation items | | | | | | | |
| Angle of curvature of film (˚) | 60 | 65 | 50 | 60 | 70 | 0 | 0 |
| Curl-up effect | ◎ | ◎ | ○ | ◎ | ◎ | × | × |

(continued)

| Composition of cosmetic (mass %) | | | | | | | |
|---|---|---|---|---|---|---|---|
| Adhesion | ○ | ◎ | ○ | ○ | ◎ | Δ | Δ |
| Note: *1: Polymethacrylic acid produced in Production Example 1;<br>*2: Sodium polystyrenesulfonate "PS-35" available from Tosoh Corporation, whose pH value was adjusted to 7 with sulfuric acid;<br>*3: Polyvinyl pyrrolidone "Polyvinyl pyrrolidone K90" available from Wako Pure Chemical Industries, Ltd. (Mw = about 360,000);<br>*4: Hydroxyethyl cellulose "QP-100" available from Union Carbide Corp. | | | | | | | |

EXAMPLES 6 TO 10 AND COMPARATIVE EXAMPLES 3 TO 6

The respective components were uniformly mixed with each other at a compositional ratio shown in Table 2 under stirring, thereby preparing mascara compositions of Examples 6 to 10 and Comparative Examples 3 to 6. The viscosity values of the thus-prepared compositions were fallen within from 100 to 2000 Pa·s as measured at 25˚C.

The mascara compositions obtained in these Examples and Comparative Examples were evaluated with respect to curl effect and formation of lumps according to the above methods (1), (3) and (4). The results are shown in Table 2.

As a result, it was confirmed that the mascara compositions obtained in Examples 6 to 10 were free from formation of lumps and exhibited a sufficient curl effect. Whereas , it was confirmed that the mascara composition obtained in Comparative Example 3 using no film-forming polymer and the mascara compositions obtained in Comparative Examples 4 and 5 using the film-forming polymers having a low contraction all exhibited neither curl effect nor effect of preventing formation of lumps, and the mascara composition obtained in Comparative Example 6 using a small amount of the shrinkable polymer in the non-volatile component exhibited only a slightly higher curl effect as compared to the case where no mascara was used.

TABLE 2-1

| | Examples | | | | |
|---|---|---|---|---|---|
| | 6 | 7 | 8 | 9 | 10 |
| Non-volatile component (A) | | | | | |
| Component (a-1) | | | | | |
| PMMA*5 ($\Delta$W = 21 mass %) | 5 | 5 | 5 | 5 | - |
| PNaSS*6 ($\Delta$W =17 mass %) | - | - | - | - | 5 |
| Wheat protein hydrolyzate*7 ($\Delta$W = 1 mass %) | - | - | - | - | - |
| Sulfoisophthalate copolymer*8 ($\Delta$W = 11 mass %) | - | - | - | - | - |
| Hydroxyethyl cellulose*9 ($\Delta$W = 1 mass %) | - | - | - | - | - |
| Carnauba wax*10 | 3 | 3 | 3 | 3 | 3 |
| Beeswax*11 | - | - | 7 | 7 | 7 |
| Extremely hydrogenated jojoba oil*12 | 6 | 6 | 6 | 6 | 6 |
| Microcrystalline wax*13 | - | - | 3 | 3 | 3 |
| Paraffin wax*14 | 9 | 9 | 4 | 4 | 4 |
| Behenyl alcohol | 1 | 1 | 1 | 1 | 1 |
| Stearic acid | 2 | 2 | 2 | 2 | 2 |
| Polyvinyl alcohol*15 | 5 | 5 | 5 | 5 | 5 |
| Water dispersion of polyethylene powder*16 | - | 2 | 2 | 2 | 2 |
| Black iron oxide | - | 8 | 8 | 8 | 8 |
| Silica*17 | - | 3 | 3 | 3 | 3 |

(continued)

| | Examples | | | | |
|---|---|---|---|---|---|
| | 6 | 7 | 8 | 9 | 10 |
| Non-volatile component (A) | | | | | |
| Component (a-1) | | | | | |
| Talc*18 | - | 1 | 1 | 1 | 1 |
| Nylon fiber*19 | 1 | 1 | 1 | 1 | 1 |
| Antiseptic agent (Methy Paraben) | 0.2 | 0.2 | 0.2 | 0.2 | 0.2 |
| Component (a-2) | | | | | |
| 1,3-butylene glycol | 5 | 5 | 5 | 5 | 5 |
| Glycerol | - | - | - | - | - |
| Polyoxyethylene glycerol monostearate*20 | 0.5 | 0.6 | 0.5 | 0.5 | 0.5 |
| Volatile component (B) | | | | | |
| 2-amino-2-methyl-1-propanol (AMP) | 1 | 1 | 1 | 1 | 1 |
| Ethanol | - | - | - | 6 | 5 |
| Purified water | Balance | Balance | Balance | Balance | Balance |
| Total | 100 | 100 | 100 | 100 | 100 |
| Amount of non-volatile component (A) | 37.7 | 50.7 | 55.7 | 55.7 | 52.7 |
| (Component (a-1)/Component (A)) x 100 | 85 | 89 | 90 | 90 | 90 |
| Evaluation of curl | 15 | 19 | 20< | 20< | 20< |
| Evaluation of formation of lumps | ○ | ○ | ○ | ◎ | ◎ |
| Contraction of bulk (angle of bend) | 22 | 28 | 30 | 35 | 34 |

TABLE 2-2

| | Comparative Examples | | | |
|---|---|---|---|---|
| | 3 | 4 | 5 | 6 |
| Non-volatile component (A) | | | | |
| Component (a-1) | | | | |
| PMMA*5 (ΔW = 21 mass %) | - | - | - | - |
| PNaSS*6 (ΔW = 17 mass %) | - | - | - | 5 |
| Wheat protein hydrolyzate*7 (ΔW = 1 mass %) | - | 5 | - | - |
| Sulfoisophthalate copolymer*8 (ΔW = 11 mass %) | - | - | 5 | - |
| Hydroxyethyl cellulose*9 (ΔW = 1 mass %) | 5 | - | - | - |
| Carnauba wax*10 | 3 | 3 | 3 | 3 |
| Beeswax*11 | 7 | 7 | 7 | 7 |
| Extremely hydrogenated jojoba oil*12 | 6 | 6 | 6 | 6 |
| Microcrystalline wax*13 | 3 | 3 | 3 | 3 |
| Paraffin wax*14 | 4 | 4 | 4 | 4 |
| Behenyl alcohol | 1 | 1 | 1 | 1 |
| Stearic acid | 2 | 2 | 2 | 2 |

(continued)

| | Comparative Examples | | | |
|---|---|---|---|---|
| | 3 | 4 | 5 | 6 |
| Non-volatile component (A) | | | | |
| Component (a-1) | | | | |
| Polyvinyl alcohol*15 | 5 | 5 | 5 | 5 |
| Water dispersion of polyethylene powder*16 | 2 | 2 | 2 | 2 |
| Black iron oxide | 8 | 8 | 8 | 8 |
| Silica*17 | 3 | 3 | 3 | 3 |
| Talc*18 | 1 | 1 | 1 | 1 |
| Nylon fiber*19 | 1 | 1 | 1 | 1 |
| Antiseptic agent (Methy Paraben) | 0.2 | 0.2 | 0.2 | 0.2 |
| Component (a-2) | | | | |
| 1,3-butylene glycol | 5 | 5 | 5 | 12 |
| Glycerol | - | - | - | 20 |
| Polyoxyethylene glycerol monostearate*20 | 0.5 | 0.5 | 0.5 | 0.5 |
| Volatile component (B) | | | | |
| 2-amino-2-methyl-1-propanol (AMP) | 1 | 1 | 1 | 1 |
| Ethanol | - | - | - | 5 |
| Purified water | Balance | Balance | Balance | Balance |
| Total | 100 | 100 | 100 | 100 |
| Amount of non-volatile component (A) | 52.7 | 48.95 | 52.7 | 79.7 |
| (Component (a-1)/Component (A)) x 100 | 90 | 89 | 90 | 51 |
| Evaluation of curl | 2 | 4 | 5 | 10 |
| Evaluation of formation of lumps | × | × | × | × |
| Contraction of bulk (angle of bend) | 0 | 0 | 3 | 10 |

Note: *5: Polymethacrylic acid produced in Production Example 1;
*6: Sodium polystyrenesulfonate "PS-35" available from Tosoh Corporation (weight-average molecular weight: 350,000; rate of change in polymer content (ΔW): 17% by mass);
*7: Wheat protein hydrolyzate "TRITISOL (solid content: 25%" available from Croda Japan KK (rate of change in polymer content (ΔW): 1% by mass);
*8: Sulfoisophthalate copolymer "EASTMAN AQ-55S" available from Eastman Chemical Company, (rate of change in polymer content (ΔW): 11% by mass);
*9: Hydroxyethyl cellulose "QP-100" available from Union Carbide Corp., (rate of change in polymer content (ΔW): 1% by mass);
*10: Carnauba wax "Purified Carnauba Wax No. 1" available from Cerarica Noda Co., Ltd. (rate of penetration: 1 or less);
*11: Beeswax "BEES WAX-S" available from Croda Japan KK (rate of penetration: 18);
*12: Extremely hydrogenated jojoba oil available from Koei Kogyo Co., Ltd. (rate of penetration: 1);
*13: Microcrystalline wax "MULTI-WAX W-445" available from Witco Corp. (rate of penetration: 34);
*14: Paraffin wax "HNP-9" available from Nippon Seiro Co., Ltd. (rate of penetration: 7);
*15: Polyvinyl alcohol "GOHSENOL EG-30" available from NIPPON GOHSEI (rate of change in polymer content (ΔW): 7% by mass);
*16: Water dispersion of polyethylene powder "Hydrocer 100 (solid content: 50%)" available from Shamlock Corp.;
*17: Silica "SUNSPHERE L31" available from Asahi Glass Co., Ltd. (average particle size: 3 μm);
*18: Talc "TALC JA-46R" available from Asada Milling Co., Ltd. (average particle size: 8 μm);
*19: Nylon fiber (length: 2 mm; fineness: 6.7T) available from UNITIKA Co., Ltd.;
*20: Polyoxyethylene glycerol monostearate "NIKKO TMGS-15" available from Nikko Chemicals Co., Ltd.

(Evaluation of Curl Effect on Eyelashes)

The mascara compositions prepared in Example 9 and Comparative Example 3 were applied to eyelashes with a brush from above 10 times and from beneath 10 times, respectively, to evaluate a curl condition of the eyelashes after drying in each case. The curl-up effect was evaluated by the same method as described above. The results are shown in Table 3.

TABLE 3

| | Curl-up effect | |
|---|---|---|
| | Applied from above 10 times | Applied from beneath 10 times |
| Example 9 | ◎ | ○ |
| Comparative Example 3 | × | × |

PRODUCTION EXAMPLE 2

20 g of the polymethacrylic acid produced in Production Example 1 was dissolved in 80 g of water. Into the resultant solution was dropped a 1N ammonia aqueous solution to adjust a pH thereof to 4.1, thereby obtaining a translucent aqueous solution of polymethacrylic acid (partial ammonium salt) having a solid content of 20% (degree of neutralization: 0.08).

Using the polymethacrylic acid obtained in Production Example 1 and the polymethacrylic acid (partial ammonium salt) obtained in Production Example 2, the following mascara compositions were formulated.

EXAMPLE 11

| | | |
|---|---|---|
| (1) | Polymethacrylic acid produced in Production Example 1 | 5% by mass |
| (2) | Beeswax "BEES WAX-S" available from Croda Japan KK (rate of penetration: 18) | 7% by mass |
| (3) | Extremely hydrogenated jojoba oil available from Koei Kogyo Co., Ltd. (rate of penetration: 1) | 6% by mass |
| (4) | Microcrystalline wax "MULTI-WAX W-445" available from Witco Corp. (rate of penetration: 34) | 3% by mass |
| (5) | Paraffin wax "HNP-9" available from Nippon Seiro Co., Ltd. (rate of penetration: 7) | 4% by mass |
| (6) | Behenyl alcohol | 1% by mass |
| (7) | Stearyl alcohol | 2% by mass |
| (8) | Polyvinyl alcohol "GOHSENOL EG-30" available from NIPPON GOHSEI (rate of change in polymer content (ΔW): 7% by mass) | 5% by mass |
| (9) | "DAITOSOL 5000AD" available from Daito Kasei Kogyo Co., Ltd. (ethyl acrylate/ethyl methacrylate copolymer emulsion; 50% water dispersion) | 5% by mass |
| (10) | Black iron oxide | 8% by mass |
| (11) | Silica "SUNSPHERE L31" available from Asahi Glass Co., Ltd. (average particle size: 3 μm) | 5% by mass |
| (12) | Talc "TALC JA-46R" available from Asada Milling Co., Ltd. (average particle size: 8 μm) | 1% by mass |
| (13) | Nylon fiber (length: 2 mm; fineness: 6.7T) available from UNITIKA Co., Ltd. | 1% by mass |
| (14) | 1,3-butylene glycol | 5% by mass |
| (15) | 2-amino-2-methyl-1-propanol (AMP) | 1% by mass |
| (16) | Polyoxyethylene glycerol monostearate "NIKKO TMGS-15" available from Nikko Chemicals Co., Ltd. | 0.5% by mass |
| (17) | Methyl parabene | 0.2% by mass |
| (18) | Ethanol | 5% by mass |
| (19) | Purified water | Balance |
| | (Total amount was adjusted to 100% by mass) | |

EXAMPLE 12

| | | |
|---|---|---|
| (1) | Polymethacrylic acid produced in Production Example 1 | 5% by mass |
| (2) | Beeswax "BEES WAX-S" available from Croda Japan KK (rate of penetration: 18) | 7% by mass |
| (3) | Extremely hydrogenated jojoba oil available from Koei Kogyo Co., Ltd. (rate of penetration: 1) | 6% by mass |
| (4) | Microcrystalline wax "MULTI-WAX W-445" available from Witco Corp. (rate of penetration: 34) | 3% by mass |
| (5) | Paraffin wax "HNP-9" available from Nippon Seiro Co., Ltd. (rate of penetration: 7) | 4% by mass |
| (6) | Behenyl alcohol | 1% by mass |
| (7) | Stearyl alcohol | 2% by mass |
| (8) | Polyvinyl alcohol "GOHSENOL EG-30" available from NIPPON GOHSEI (rate of change in polymer content (ΔW): 7% by mass) | 5% by mass |
| (9) | "DAITOSOL 5000AD" available from Daito Kasei Kogyo Co., Ltd. (ethyl acrylate/ethyl methacrylate copolymer emulsion; 50% water dispersion) | 5% by mass |
| (10) | 3% polyacrylic acid-treated black iron oxide available from Miyoshi Kasei Co., Ltd. | 8% by mass |
| (11) | Silica "SUNSHERE L31" available from Asahi Glass Co., Ltd. (average particle size: 3 μm) | 5% by mass |
| (12) | Talc "TALC JA-46R" available from Asada Milling Co., Ltd. (average particle size: 8 μm) | 1% by mass |
| (13) | Nylon fiber (length: 2 mm; fineness: 6.7T) available from UNITIKA Co., Ltd. | 1% by mass |
| (14) | 1,3-butylene glycol | 5% by mass |
| (15) | 2-amino-2-methyl-1-propanol (AMP) | 1% by mass |
| (16) | Polyoxyethylene glycerol monostearate "NIKKO TMGS-15" available from Nikko Chemicals Co., Ltd. | 0.5% by mass |
| (17) | Methyl parabene | 0.2% by mass |
| (18) | Ethanol | 5% by mass |
| (19) | Purified water | Balance |

(Total amount was adjusted to 100% by mass)

**EXAMPLE 13**

| | | |
|---|---|---|
| (1) | Polymethacrylic acid produced in Production Example 2 | 5% by mass |
| (2) | Beeswax "BEES WAX-S" available from Croda Japan KK (rate of penetration: 18) | 5% by mass |
| (3) | Extremely hydrogenated jojoba oil available from Koei Kogyo Co., Ltd. (rate of penetration: 1) | 6% by mass |
| (4) | Paraffin wax "HNP-9" available from Nippon Seiro Co., Ltd. (rate of penetration: 7) | 4% by mass |
| (5) | Behenyl alcohol | 1% by mass |
| (6) | Stearyl alcohol | 2% by mass |
| (7) | Polyvinyl alcohol "GOHSENOL EG-30" available from NIPPON GOHSEI (rate of change in polymer content (ΔW): 7% by mass) | 5% by mass |
| (8) | "DAITOSOL 5000AD"; polymer emulsion available from Daito Kasei Kogyo Co., Ltd. | 5% by mass |
| (9) | Black iron oxide | 8% by mass |
| (10) | Silica "SUNSPHERE L31" available from Asahi Glass Co., Ltd. (average particle size: 3 μm) | 2% by mass |
| (11) | Talc "TALC JA-46R" available from Asada Milling Co., Ltd. (average particle size: 8 μm) | 0.5% by mass |
| (12) | Nylon fiber (length: 2 mm; fineness: 6.7T) available from UNITIKA Co., Ltd. | 1% by mass |
| (13) | 1,3-butylene glycol | 5% by mass |
| (14) | 2-amino-2-methyl-1-propanol (AMP) | 1% by mass |
| (15) | Polyoxyethylene glycerol monostearate "NIKKO TMGS-15" available from Nikko Chemicals Co., Ltd. | 0.5% by mass |
| (16) | Methyl parabene | 0.2% by mass |
| (17) | Ethanol | 1% by mass |

(continued)

| | | |
|---|---|---|
| (18) | Purified water | Balance |

(Total amount was adjusted to 100% by mass)

The mass ratio of the film-forming polymer (polymethacrylic acid) to the wax having a rate of penetration of 8 or more was 5/4; the mass ratio of the film-forming polymer to the powder having a particle size of 1 μm or more was 2/1; and the mass ratio of the film-forming polymer to ethanol was 5/1.

EXAMPLE 14

| | | |
|---|---|---|
| (1) | Polymethacrylic acid produced in Production Example 1 | 2% by mass |
| (2) | Beeswax "BEES WAX-S" available from Croda Japan KK (rate of penetration: 18) | 7% by mass |
| (3) | Extremely hydrogenated jojoba oil available from Koei Kogyo Co., Ltd. (rate of penetration: 1) | 6% by mass |
| (4) | Microcrystalline wax "MULTI-WAX W-445" available from Witco Corp. (rate of penetration: 34) | 3% by mass |
| (5) | Paraffin wax "HNP-9" available from Nippon Seiro Co., Ltd. (rate of penetration: 7) | 4% by mass |
| (6) | Behenyl alcohol | 1% by mass |
| (7) | Stearyl alcohol | 2% by mass |
| (8) | Polyvinyl alcohol "GOHSENOL EG-30" available from NIPPON GOHSEI (rate of change in polymer content (ΔW): 7% by mass) | 5% by mass |
| (9) | "DAITOSOL 5000AD" available from Daito Kasei Kogyo Co., Ltd. (ethyl acrylate/ethyl methacrylate copolymer emulsion; 50% water dispersion) | 5% by mass |
| (10) | 3% polyacrylic acid-treated black iron oxide available from Miyoshi Kasei, Inc. | 8% by mass |
| (11) | Silica "SUNSHERE L31" available from Asahi Glass Co., Ltd. (average particle size: 3 μm) | 3% by mass |
| (12) | Talc "TALC JA-46R" available from Asada Milling Co., Ltd. (average particle size: 8 μm) | 1% by mass |
| (13) | Nylon fiber (length: 2 mm; fineness: 6.7T) available from UNITIKA Co., Ltd. | 1% by mass |
| (14) | 1,3-butylene glycol | 5% by mass |
| (15) | 2-amino-2-methyl-1-propanol (AMP) | 1% by mass |
| (16) | Methyl parabene | 0.2% by mass |
| (17) | Ethanol | 8% by mass |
| (18) | Purified water | Balance |

(Total amount was adjusted to 100% by mass)

The mass ratio of the film-forming polymer (polymethacrylic acid) to the wax having a rate of penetration of 8 or more was 2/7; the mass ratio of the film-forming polymer to the powder having a particle size of 1 μm or more was 2/1; and the mass ratio of the film-forming polymer to ethanol was 1/4.

INDUSTRIAL APPLICABILITY

The cosmetic of the present invention can be suitably used as a make-up cosmetic for eyelashes, more specifically, as mascara, and can impart a good curl effect to eyelashes without formation of lumps.

## Claims

**1.** A cosmetic for eyelash comprising a film-forming polymer (a) having a rate of change in polymer content (ΔW) as defined below of 13% or more, and water, a content of the film-forming polymer (a) in the cosmetic being from 0.6 to 50% by mass:

Definition of Rate of Change in Polymer Content (ΔW): Film-Forming Test
ΔW is a rate of change in polymer content which is caused during a film-forming process in which a film obtained from the polymer which has a pencil hardness of 2B is dried up, said ΔW being represented by the following

formula (I):

$$\Delta W = W2 - W1 \qquad (I)$$

wherein W1 and W2 are respectively defined as follow:

An aqueous solution containing 20% by mass of the polymer is applied onto two glass plates over an area of 11 cm x 20 cm thereof using an applicator to form a coating film having 250 μm, and then dried at a temperature of 23°C and a relative humidity (RH) of 65%; and the first coating film formed by spreading the solution on one glass plate is subjected to measurement of change in weight thereof upon drying, and the second coating film formed on another glass plate is subjected to measurement of a hardness thereof by a pencil hardness measuring method to calculate a polymer content W1 (%) upon drying the coating film into a pencil hardness of 2B according to the following formula, and further the coating film is allowed to stand for 24 hours after coating (hereinafter referred to as "after drying up") to calculate a polymer content W2 (%) after drying up according to the following formula:

$$W1\ (\%) = (Wp/W1t) \times 100$$

$$W2\ (\%) = (Wp/W2t) \times 100$$

wherein Wp represents a weight of the polymer in the coating film upon coating; W1t represents a weight of the coating film upon drying into a pencil hardness of 2B; and W2t is a weight of the coating film after drying up.

**2.** The cosmetic for eyelash according to claim 1, wherein the cosmetic comprises (A) 10% by mass or more of a non-volatile component and (B) 30% by mass or more of a volatile component, said non-volatile component (A) comprising (a-1) 70% by mass or more of a non-volatile component kept in a solid state at ordinary temperature which contains the film-forming polymer (a), and (a-2) 30% by mass or less of a non-volatile component kept in a liquid state at ordinary temperature.

**3.** The cosmetic for eyelash according to claim 2, wherein the film-forming polymer (a) has a weight-average molecular weight of 5,000 to 2,000,000.

**4.** The cosmetic for eyelash according to claim 3, wherein a content of the film-forming polymer (a) is 1 to 15% by mass on the basis of a total amount of the cosmetic.

**5.** The cosmetic for eyelash according to any one of claims 1 to 4, wherein the film-forming polymer (a) is a polymer obtained by polymerizing a vinyl monomer.

**6.** The cosmetic for eyelash according to any one of claims 1 to 5, wherein the film-forming polymer (a) is a polymer obtained by polymerizing a monomer containing a reactive vinyl group-containing organic acid and/or a salt thereof.

**7.** The cosmetic for eyelash according to claim 6, wherein the reactive vinyl group-containing organic acid is at least one organic acid selected from the group consisting of methacrylic acid and styrenesulfonic acid.

**8.** The cosmetic for eyelash according to claim 6 or 7, wherein a whole or part of a carboxylic acid or a sulfonic acid contained in the film-forming polymer (a) is neutralized with ammonia.

**9.** The cosmetic for eyelash according to any one of claims 2 to 8, wherein the component (A) further comprises a wax having a rate of penetration of 8 or more.

**10.** The cosmetic for eyelash according to any one of claims 2 to 9, wherein the component (A) further comprises a powder having an average particle size of 0.1 to 20 μm.

**11.** The cosmetic for eyelash according to any one of claims 2 to 10, wherein the volatile component (B) contains water.

**12.** The cosmetic for eyelash according to any one of claims 2 to 11, wherein the volatile component (B) contains water and a lower alcohol having 1 to 4 carbon atoms to form an oil-in-water type emulsion composition.

**13.** The cosmetic for eyelash according to any one of claims 1 to 12, wherein when 0.04 g of the cosmetic is applied onto a polyethylene terephthalate film having a width of 2 cm, a length of 5 cm and a thickness of 75 μm to form a coating film having a width of 4 mm in a length direction of the polyethylene terephthalate film and a length of 2 cm in a width direction of the polyethylene terephthalate film on a central portion of the length direction of the polyethylene terephthalate film, and dried at a temperature of 23°C and a relative humidity of 60% for 12 hours, the coated polyethylene terephthalate film exhibit an angle of bend of 5° or more.

**14.** A method of using the cosmetic for eyelash as defined in any one of claims 1 to 13, comprising allowing a larger amount of the cosmetic to adhere onto an upper surface of the eyelash.

**INTERNATIONAL SEARCH REPORT**

International application No.
PCT/JP2005/005019

A. CLASSIFICATION OF SUBJECT MATTER
Int.Cl$^7$ A61K7/032

According to International Patent Classification (IPC) or to both national classification and IPC

B. FIELDS SEARCHED

Minimum documentation searched (classification system followed by classification symbols)
Int.Cl$^7$ A61K7/032

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2005 |
| Kokai Jitsuyo Shinan Koho | 1971-2005 | Toroku Jitsuyo Shinan Koho | 1994-2005 |

Electronic data base consulted during the international search (name of data base and, where practicable, search terms used)
CAPLUS(STN)

C. DOCUMENTS CONSIDERED TO BE RELEVANT

| Category* | Citation of document, with indication, where appropriate, of the relevant passages | Relevant to claim No. |
|---|---|---|
| X<br>Y | JP 11-255619 A (L'Oreal),<br>21 September, 1999 (21.09.99),<br>Claims; Par. No. [0015]<br>& BR 9805679 A & CA 2256065 A1<br>& CN 1227096 A & DE 69800751 A<br>& EP 0928607 A1 & ES 2158654 A<br>& FR 2773063 A1 & PL 330592 A1<br>& US 6274131 B1 & US 2002/004036 A1 | 1-4,11-14<br>5-10 |
| Y | JP 06-009341 A (L'Oreal),<br>18 January, 1994 (18.01.94),<br>Column 8, lines 38 to 40; column 9, line 22;<br>Par. No. [0076]<br>& AT 132360 A & CA 2089984 A1<br>& DE 69301166 A & EP 0557196 A1<br>& ES 2082598 A & FR 2687569 A1<br>& US 5849278 A & US 5858338 A | 5-8,10 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 17 June, 2005 (17.06.05) | 05 July, 2005 (05.07.05) |
| Name and mailing address of the ISA/<br>Japanese Patent Office<br>Facsimile No. | Authorized officer<br><br>Telephone No. |

Form PCT/ISA/210 (second sheet) (January 2004)

INTERNATIONAL SEARCH REPORT

International application No.
PCT/JP2005/005019

C (Continuation). DOCUMENTS CONSIDERED TO BE RELEVANT

| Category* | Citation of document, with indication, where appropriate, of the relevant passages | Relevant to claim No. |
|---|---|---|
| Y | JP 05-194148 A (L'Oreal), 03 August, 1993 (03.08.93), Claim 1 & AT 135563 A & DE 69209194 A & DK 530084 A & EP 0530084 A1 & ES 2084307 A & FR 2680681 A1 & GR 3019890 A | 9 |
| Y | JP 2003-055156 A (L'Oreal), 26 February, 2003 (26.02.03), Par. Nos. [0116] to [0118] & EP 1281384 A1 & FR 2827168 A1 & US 2003/059388 A1 | 9 |

Form PCT/ISA/210 (continuation of second sheet) (January 2004)

INTERNATIONAL SEARCH REPORT

International application No.
PCT/JP2005/005019

Claim 1 involves a cosmetic preparation for eyelashes which contains a film-forming polymer having the desired property of "having a polymer content change (ΔW) of 13% or larger." However, the film-forming polymer which is disclosed in the meaning of Article 5 of the PCT is limited to the specific polymers given in the description, i.e., the polymethacrylic acid described in Production Examples 1 and 2 and the sodium polystyrenesulfonate specified by a trade name (PS-35) (Tosoh Corp.). Therefore, claim 1 lacks a support in the meaning of Article 6 of the PCT.

Form PCT/ISA/210 (extra sheet) (January 2004)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 11256619 A
- JP 2003055136 A
- JP 2003055156 A
- JP 2003055157 A
- JP 2003055158 A